# EUROPÄISCHE PATENTSCHRIFT

(11) **EP 3 381 489 B1**
(45) Veröffentlichungstag und Bekanntmachung des Hinweises auf die Patenterteilung: **29.04.2020**
(21) Anmeldenummer: 18161566.7
(22) Anmeldetag: 13.03.2018
(51) Int. Cl.: A61M 5/168, A61M 5/152, A61M 5/145

(54) **MEDIZINISCHE PUMPVORRICHTUNG**
MEDICAL PUMPING DEVICE
DISPOSITIF DE POMPAGE MÉDICAL

(30) Priorität: 28.03.2017 DE 102017205251
(43) Veröffentlichungstag der Anmeldung: 03.10.2018
(73) Patentinhaber: B. Braun Melsungen AG, 34212 Melsungen (DE)
(72) Erfinder: Stettner, Jens, 34212 Melsungen (DE)
(74) Vertreter: Patentanwälte Ruff, Wilhelm, Beier, Dauster & Partner mbB

(56) Entgegenhaltungen:
- DE-A1- 19 840 965
- DE-A1-102013 111 800
- US-A- 3 993 069
- US-A1- 2013 053 775

## Beschreibung

Die Erfindung betrifft eine medizinische Pumpvorrichtung zur Förderung eines medizinischen Fluids mit einem Grundkörper und einer an dem Grundkörper festgelegten elastomeren Hohlmembran, die derart angeordnet ist, dass zwischen dem Grundkörper und der Hohlmembran ein Pumpvolumen zur Befüllung mit dem medizinischen Fluid gebildet ist, wobei die Hohlmembran in einem zumindest teilweise befüllten Füllzustand des Pumpvolumens elastisch gedehnt ist und derart einen Förderdruck auf das Pumpvolumen bewirkt.

Eine derartige medizinische Pumpvorrichtung ist im Bereich der Medizintechnik allgemein bekannt und kann bei der Verabreichung von medizinischen Fluiden im Rahmen einer ambulanten Infusionstherapie verwendet werden. Solche medizinischen Pumpvorrichtungen, die auch als Elastomerpumpen oder elastomerische Pumpsysteme bezeichnet werden, weisen einen Grundkörper sowie eine an dem Grundkörper festgelegte elastomere Hohlmembran auf. Die elastomere Hohlmembran weist gummielastische Dehnungseigenschaften auf und kann nach Art eines Ballons oder einer Blase ausgebildet sein. Die Hohlmembran bildet derart eine äußere Berandung eines Pumpvolumens, das zur Aufnahme und Abgabe des medizinischen Fluids vorgesehen ist. Bei der Befüllung des Pumpvolumens mit dem Fluid wird die elastomere Hohlmembran elastisch gedehnt, wobei deren Oberfläche vergrößert und folglich mechanische Energie in der Hohlmembranwandung gespeichert wird. Die derart elastisch gedehnte Hohlmembran bewirkt einen Förderdruck auf das Pumpvolumen. Durch diesen Förderdruck kann das medizinische Fluid aus dem Pumpvolumen in eine der Pumpvorrichtung nachgelagerte Schlauchleitung und weiter in einen mit dieser verbindbaren patientenseitigen Zugang gefördert werden. Je nach Spezifikation und Anwendungsfall der Pumpvorrichtung kann die Förderdauer hierbei wenige Minuten bis mehrere Tage betragen. Um die bereits geförderte Fluidmenge und unter Umständen eine Fehlfunktion der Pumpvorrichtung erkennen zu können, ist es notwendig, während der Förderdauer eine Kontrolle des Füllstandes des Pumpvolumens vorzunehmen. Üblicherweise wird die Pumpvorrichtung hierzu vor und während der Förderdauer mittels einer Waage gewogen oder die Hohlmembran wird mit einem Maßband vermessen. Die derart ermittelten Messwerte werden dokumentiert. Ausgehend von diesen kann sodann auf den aktuellen Füllzustand des Pumpvolumens geschlossen werden.

Die US 3 993 069 A lehrt eine Vorrichtung gemäß dem Oberbegriff des Anspruchs 1.

Aufgabe der Erfindung ist es, eine medizinische Pumpvorrichtung der eingangs genannten Art zu schaffen, die eine verbesserte Anwendungssicherheit und Handhabbarkeit gewährleistet.

Diese Aufgabe wird durch eine Vorrichtung gemäß Anspruch 1 gelöst. Durch die erfindungsgemäße Lösung ist es möglich, auf ein externes Messinstrument zur Ermittlung des Füllzustandes zu verzichten. Dies erlaubt zum einen die Reduzierung von Messfehlern, da Anwenderfehler beim Ablesen des externen Messinstrumentes und Fehler bei der Dokumentation und Verarbeitung der Messwerte ausgeschlossen werden. Zum anderen wird demzufolge eine bessere Anwenderfreundlichkeit der Pumpvorrichtung insbesondere im ambulanten Anwendungsumfeld erreicht, da solche externen Messinstrumente durch den Anwender weder vorgehalten noch mitgeführt werden müssen. Hierdurch ermöglicht die erfindungsgemäße Lösung eine erhöhte Anwendungssicherheit und Handhabbarkeit der medizinischen Pumpvorrichtung. Die Ermittlung der Dehnung der elastomeren Hohlmembran kann hierbei unmittelbar oder mittelbar erfolgen. Zur unmittelbaren Erfassung der Dehnung kann das Sensoriksystem zur Erfassung einer Längenänderung zumindest eines Abschnittes einer Oberfläche der Hohlmembran eingerichtet sein. Zur mittelbaren Erfassung der Dehnung kann das Sensoriksystem zur Erfassung einer Kraft, eines Druckes, einer Distanz oder einer optischen Messgröße eingerichtet sein. Unter einer elastomeren Hohlmembran im Sinne der Erfindung ist eine Membran zu verstehen, die gummielastische Dehnungseigenschaften aufweist und im Wesentlichen nach Art einer Blase oder eines Ballons ausgebildet ist. Das Material der elastomeren Hohlmembran kann Silikon und/oder Kautschuk und/oder einen anderen gummielastischen Werkstoff beinhalten. Vorteilhafterweise beinhaltet das Material der elastomeren Hohlmembran im Wesentlichen Silikon. Vorteilhafterweise ist das medizinische Pumpsystem derart ausgestaltet, dass es am Körper eines Anwenders durch diesen ohne weiteres transportabel ist. Es ist vorteilhaft, wenn das Sensoriksystem im Wesentlichen innerhalb eines Gehäuses der Pumpvorrichtung, insbesondere im Wesentlichen innerhalb des Grundkörpers, angeordnet ist. Derart wird eine besonders kompakte und besonders leicht handhabbare Pumpvorrichtung erreicht.

In Ausgestaltung der Erfindung weist das Sensoriksystem eine Sensoranordnung auf, die an der Hohlmembran angeordnet und mit dieser verbundenen ist. Die Sensoranordnung kann zumindest einen Sensor umfassen. Der Sensor kann auf einem resistiven, kapazitiven oder induktiven Messprinzip basieren. Die Verbindung zwischen der Sensoranordnung und der elastomeren Hohlmembran kann eine Klebeverbindung sein. Vorteilhafterweise ist die Verbindung zwischen der Hohlmembran und der Sensoranordnung derart gestaltet, dass eine Dehnung zumindest eines Abschnittes der elastomeren Hohlmembran eine in Art und Umfang entsprechende Dehnung zumindest eines Abschnittes der Sensoranordnung bewirkt. Auf diese Weise wird eine besonders bauraumsparende Ausgestaltung der Erfindung erreicht.

In weiterer Ausgestaltung der Erfindung sind die Sensoranordnung und die Hohlmembran mittels eines Koextrusionsverfahrens miteinander verbunden. Extrusionsverfahren sind als solche allgemein bekannt. Bei solchen Verfahren wird eine auszuformende Masse unter Druckbeaufschlagung kontinuierlich durch eine formgebende Öffnung gepresst und bildet derart ein Extrudat. Bei Koextrusionsverfahren werden dementsprechend mindestens zwei Massen gleichzeitig durch ein und dieselbe Öffnung extrudiert und bilden derart ein fest miteinander verbundenes Koextrudat. Vorteilhafterweise sind die Sensoranordnung und die Hohlmembran in Gestalt eines Schlauchabschnittes koextrudiert, wobei zumindest ein Abschnitt der Sensoranordnung entlang der Längsrichtung des Schlauches erstreckt und auf oder in einer Umfangsfläche des Schlauches angeordnet ist. Vorteilhafterweise umfasst die Sensoranordnung zwei Streifen leitfähigen Materials, die an oder in einer Umfangsfläche des Koextrudats in etwa 180 Grad winkelversetzt zueinander angeordnet sind. Durch die derartige Koextrusion werden zum einen besonders vorteilhafte Materialeigenschaften hinsichtlich der Festigkeit der Verbindung zwischen der Hohlmembran und der Sensoranordnung erreicht. Zum anderen ist diese Ausgestaltung besonders kostengünstig herstellbar.

In weiterer Ausgestaltung der Erfindung weist die Sensoranordnung zumindest einen Dehnungsmessstreifen auf. Das einem Dehnungsmessstreifen zugrundeliegende resistive Messprinzip ist allgemein bekannt. Es basiert auf der dehnungsabhängigen Änderung des elektrischen Widerstandes des Dehnungsmessstreifens. Insoweit wird die Änderung des elektrischen Widerstands des Dehnungsmessstreifens ermittelt, aus dieser auf die Längenänderung und somit auf die Dehnung des Dehnungsmessstreifens sowie der mit diesem verbundenen Hohlmembran geschlossen. Aus der derart ermittelten Dehnung der Hohlmembran ist letztlich die Füllstandsinformation ermittelbar. Vorteilhafterweise ist der Dehnungsmessstreifen zumindest abschnittsweise durch einen mit der Hohlmembran koextrudierten leitfähigen Werkstoff gebildet. Alternativ oder zusätzlich kann der Dehnungsmesstreifen zumindest abschnittsweise aus einem auf oder in die Hohlmembran geklebten leitfähigen Material gebildet sein. Der Dehnungsmessstreifen erstreckt sich vorteilhafterweise entlang der Längsrichtung der Hohlmembran. Alternativ oder zusätzlich kann der Dehnungsmessstreifen entlang der Umfangsrichtung der Hohlmembran verlaufen. Um eine besonders hohe Messempfindlichkeit zu erreichen, kann der Dehnungsmessstreifen vorteilhafterweise an der in gefülltem Zustand des Pumpvolumens in radialer Richtung am stärksten gedehnten Stelle der Hohlmembran in Umfangsrichtung erstreckt angeordnet sein.

Gemäß der Erfindung weist das Sensoriksystem eine elektronische Auswerteeinheit auf, die derart eingerichtet ist, dass ein mittels des Sensoriksystems ermitteltes Messsignal in einen Messwert umsetzbar ist, ein Differenzenzwert zwischen dem Messwert und einem Referenzwert ermittelbar ist und die Füllstandsinformation unter Verwendung des Differenzwertes ermittelbar ist. Vorteilhafterweise ist die Auswerteeinheit mit der Sensoranordnung verbunden und zur Umsetzung eines Spannungsmesssignals in einen Dehnungsmesswert eingerichtet. Der Referenzwert ist vorteilhafterweise in einer Speichereinheit der Auswerteeinheit hinterlegt. Vorteilhafterweise ist der Referenzwert ein Dehnungsmesswert, der in einem im Wesentlichen vollständig gefüllten oder entleerten Füllzustand des Pumpvolumens ermittelt wurde. Eine besonders genaue Ermittlung des Füllstandes wird erreicht, wenn mehrere Referenzwerte in Gestalt einer Referenzkurve hinterlegt sind. Demzufolge kann aus dem Differenzwert zwischen dem Messwert und dem Referenzwert auf den Füllstand des Pumpvolumens geschlossen und letztlich eine Füllstandsinformation ermittelt werden. Die Füllstandsinformation kann vorteilhafterweise als relative Größe in Bezug auf die Füllmenge zu Beginn der Förderdauer ermittelt werden. Alternativ oder zusätzlich kann die Füllstandsinformation als relative Größe in Bezug auf einen entleerten Zustand des Pumpvolumens ermittelt werden. Da derart Fehler bei der Auswertung von Messwerten durch einen Anwender vermieden werden, wird eine besonders anwendungssichere Ausgestaltung der Erfindung erreicht.

In weiterer Ausgestaltung der Erfindung weist das Sensoriksystem eine Ausgabeeinheit auf, die zur Ausgabe der Füllstandsinformation eingerichtet ist. Vorteilhafterweise kann die Ausgabeeinheit mit der Auswerteeinheit verbunden sein. Die Füllstandsinformation kann optisch oder akustisch oder in einen anderen anwenderseitig wahrnehmbaren Art und Weise ausgegeben werden. Demzufolge erhält ein Anwender der Pumpvorrichtung die Füllstandsinformation auf besonders verlässliche und leicht erfassbare Art und Weise.

In weiterer Ausgestaltung der Erfindung weist die Ausgabeeinheit eine Anzeigeeinheit auf, die zur optischen Darstellung der Füllstandsinformation eingerichtet ist. Die Anzeigeeinheit kann vorteilhafterweise ein Display umfassen. Das Display kann mit dem Gehäuse der Pumpvorrichtung, insbesondere mit dem Grundkörper, verbunden sein. Diese Ausgestaltung der Erfindung ermöglicht eine besonders komfortable Erfassung der Füllstandsinformation durch einen Anwender oder eine medizinisch geschulte Person.

In weiterer Ausgestaltung der Erfindung weist die Ausgabeeinheit eine Übertragungseinheit auf, die zur kabellosen Übertragung der Füllstandsinformation eingerichtet ist. Die Übertragung der Füllstandsinformation kann in Gestalt eines Signals an eine externe Anzeige- oder Wiedergabeeinheit, insbesondere ein Smartphone, erfolgen. Derart wird eine alternative oder zusätzliche Einsehbarkeit der Füllstandsinformation durch eine von dem Anwender örtlich getrennte Instanz, beispielsweise eine medizinisch geschulte Person, ermöglicht. Demzufolge wird eine besonders anwendungssichere Ausgestaltung der Erfindung erreicht.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.
- Fig. 1: zeigt in perspektivischer Darstellung eine bevorzugte Ausführungsform einer erfindungsgemäßen medizinischen Pumpvorrichtung mit einem Sensoriksystem,
- Fig. 2: in schematischer Darstellung der medizinischen Pumpvorrichtung gemäß Fig. 1 in einem ungefüllten Füllzustand des Pumpvolumens,
- Fig. 3: in schematischer Darstellung die Pumpvorrichtung gemäß Fig. 1 und Fig. 2 in einem gefüllten Füllzustand des Pumpvolumens und
- Fig. 4: in schematischer Darstellung einen Teil der Pumpvorrichtung gemäß Fig. 1 bis 3 im Bereich der Hohlmembran und des Sensoriksystems.

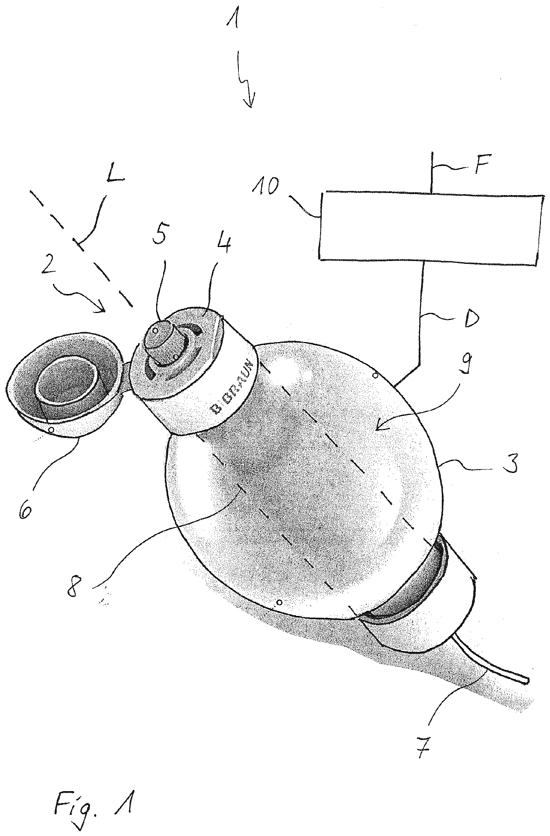

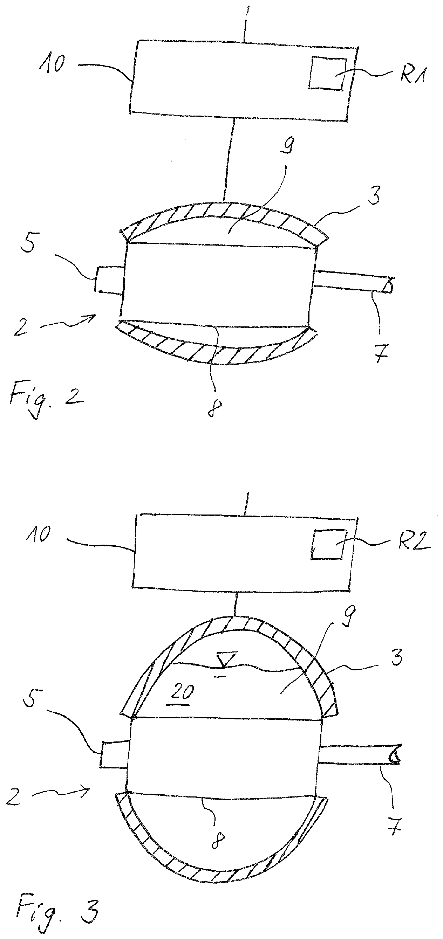

Eine medizinische Pumpvorrichtung 1 nach Fig. 1 bis 4 ist zur Förderung eines medizinischen Fluids im Rahmen einer ambulanten Infusionstherapie vorgesehen. Zu diesem Zweck weist die medizinische Pumpvorrichtung 1 einen Grundkörper 2 und eine an dem Grundkörper 2 festgelegte elastomere Hohlmembran 3 auf. Der Grundkörper 2 weist ein Befüllventil 4, eine Ventilkappe 5 sowie eine Verschlusskappe 6 auf, die jeweils eingangsseitig und axial zur Längsachse L des Grundkörpers 2 angeordnet sind, was jedoch nicht zwingend ist. Grundsätzlich können das Befüllventil 4 sowie die Ventilkappe 5 und die Verschlusskappe 6 von dem Grundkörper 2 entkoppelt angeordnet sein. An der Ausgangsseite des Grundkörpers 2 ist ein Infusionsschlauch 7 angeordnet, welcher nicht notwendigerweise Bestandteil der medizinischen Pumpvorrichtung 1 ist. In einer nicht näher dargestellten Art und Weise können dem Infusionsschlauch 7 eine Verschlussklemme, ein luftabscheidender Partikelfilter, ein Durchflussbegrenzer sowie eine Anschlusskonnektor zur Anbindung an einen patientenseitigen Zugang zugeordnet sein. Die elastomere Hohlmembran 3 ist nach Art eines Ballons ausgebildet und umschließt einen in Fig. 1 strichliert dargestellten Mittelteil 8 des Grundkörpers 2 im Wesentlichen koaxial zu dessen Längsachse L. Zwischen dem Mittelteil 8 des Grundkörpers 2 und der elastomeren Hohlmembran 3 ist ein Pumpvolumen 9 zur Befüllung mit einem anhand Fig. 3 schematisch ersichtlichen medizinischen Fluid 20 ausgebildet. Zur Befüllung des Pumpvolumens 9 mit dem medizinischen Fluid 20 wird die Verschlusskappe 6 in einen anhand Fig. 1 ersichtlichen geöffneten Zustand gebracht, die Ventilkappe 5 wird gelöst und das medizinische Fluid 20 wird über das Befüllventil 4 in den zwischen der elastomeren Hohlmembran 3 und dem Mittelteil 8 des Grundkörpers 2 gebildeten Zwischenraum, in anderen Worten in das Pumpvolumen 9, eingefüllt. Die elastomere Hohlmembran 3 besteht aus SilikonElastomer und ist insoweit gummielastisch dehnbar. Bei der derartigen Befüllung des Pumpvolumens 9 mit dem Fluid 20 vergrößert sich daher die Oberfläche der elastomeren Hohlmembran 3 und speichert folglich mechanische Energie. Die auf die Weise gummielastisch gedehnte Hohlmembran 3 bewirkt einen Förderdruck auf das Pumpvolumen 9. Nach dem Befüllen des Pumpvolumens 9 wird die Ventilkappe 5 geschlossen und die Verschlussklappe 6 wird in einen verschlossenen Zustand gebracht. Nach Öffnen der dem Infusionsschlauch 7 zugeordneten und mit diesem in einer Wirkverbindung stehenden Verschlussklemme kann das medizinische Fluid 20 infolge des mittels der elastischen Hohlmembran 3 bewirkten Förderdruckes längs des Infusionsschlauches 7 bis zu dem patientenseitigen Zugang gefördert werden.

Zur Ermittlung einer Füllstandsinformation F zum Füllzustand des Pumpvolumens 9 weist die medizinische Pumpvorrichtung zudem ein Sensoriksystem 10 auf, dass zur Ermittlung der Dehnung D der elastomeren Hohlmembran 3 vorbereitet ist. Aus Gründen der Übersichtlichkeit ist das Sensoriksystem 10 anhand der Fig. 1 bis 4 lediglich schematisch dargestellt und in seiner funktionellen Beziehung zu den übrigen Komponenten der Pumpvorrichtung 1 ersichtlich.

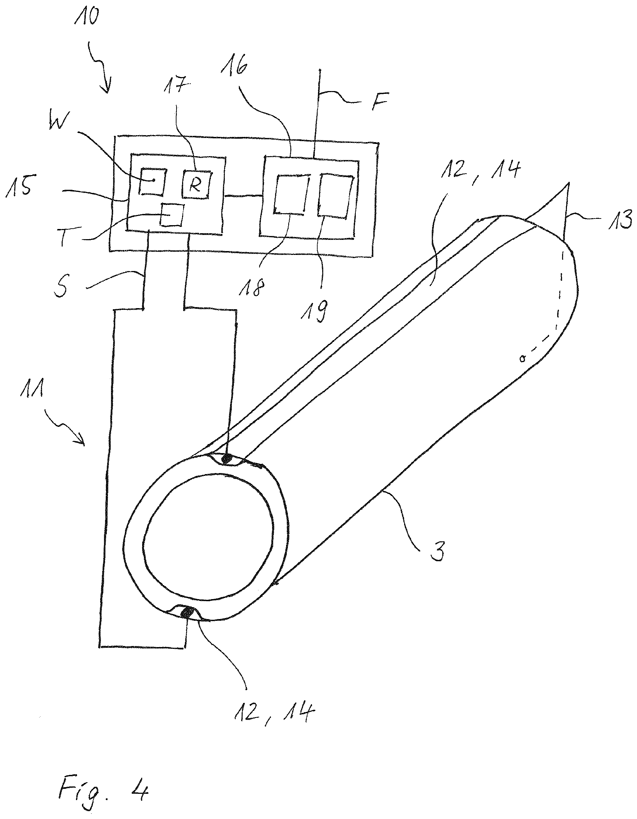

Wie anhand Fig. 4 ersichtlich ist, weist das Sensoriksystem 10 eine an der elastomeren Hohlmembran 3 angeordnete und mit dieser verbundene Sensoranordnung 11 auf. Die elastomere Hohlmembran 3 ist in Fig. 4 in einem unmontierten und von daher im Wesentlichen ungedehnten, schlauchartigen Zustand dargestellt. Die Sensoranordnung 11 weist zwei jeweils mit der Außenfläche der Hohlmembran 3 verbundene in Längsrichtung der Hohlmembran 3 erstreckte Streifen 12 auf. Die Streifen 12 weisen eine elektrisch leitfähige Materialzusammensetzung auf. Vorzugsweise umfassen die Streifen 12 eine metallische Materialzusammensetzung. Besonders bevorzugt weist die Materialzusammensetzung ein Polymer-Metall- oder ein Polymer-Graphit-Gemisch oder ein organisches Halbleitermaterial auf. Die Streifen 12 sind mittels eines Koextrusionsverfahrens mit der Hohlmembran 3 verbunden und weisen an einer Stirnseite der Hohlmembran eine elektrisch leitfähige Verbindung 13 auf. Bei dieser Art der Sensoranordnung 11 ändert sich bei einer Längenänderung der Hohlmembran 3 die elektrische Leitfähigkeit und damit der elektrische Widerstand der Streifen 12. Die Streifen 12 bilden derart einen Dehnungsmessstreifen 14 aus, mit dem die Dehnung D der elastomeren Hohlmembran 3 ermittelt werden kann.

Zu diesem Zweck weist das Sensoriksystem 10 zudem eine elektronische Auswerteeinheit 15 und eine Ausgabeeinheit 16 auf, wie anhand Fig. 4 näher ersichtlich ist. Die elektronische Auswerteeinheit 15 ist mit der Sensoranordnung 11, genauer: stirnseitig mit den Streifen 12, elektrisch leitfähig verbunden. Auf diese Weise ist ein mittels der Sensoranordnung 11 ermitteltes Messsignal S in Form eines Spannungssignals an dem durch die Streifen 12 gebildeten Dehnungsmessstreifen 14 abgreifbar. Die Auswerteeinheit 15 ist zudem dazu eingerichtet, das derart ermittelte Messsignal S in einen Messwert W umzusetzen und einen Differenzwert T zwischen dem Messwert W und einem Referenzmesswert R, der in einer Speichereinheit 17 der Auswerteeinheit 15 hinterlegt ist, zu ermitteln. Die Ausgabeeinheit 16 ist zur Ausgabe der Füllstandsinformation F eingerichtet und weist zu diesem Zweck eine Anzeigeeinheit 18 und alternativ oder zusätzlich eine Übertragungseinheit 19 auf. Die Anzeigeeinheit 18 ist zur optischen Darstellung der Füllstandsinformation F eingerichtet und in Form eines Displays gestaltet. Die Übertragungseinheit 19 ist zur kabellosen Übertragung der Füllstandsinformation F eingerichtet und erlaubt derart eine Übertragung der Füllstandsinformation F auf eine nicht näher dargestellte von der Pumpvorrichtung 1 getrennte Anzeige- oder Wiedergabeeinheit.

Die Ermittlung der Füllstandsinformation F zum Füllstand des Pumpvolumens 9 unter Verwendung der medizinischen Pumpvorrichtung 1 kann, wie nachfolgend dargelegt, erfolgen: In einem anhand Fig. 2 schematisch ersichtlichen unbefüllten Zustand der Pumpvorrichtung 1 wird mittels des Sensoriksystems 10 ein erster Referenzmesswert R1 in Form eines Dehnungswertes des ungedehnten Zustandes der Hohlmembran 3 ermittelt. Dieser Referenzmesswert R1 kann in der Speichereinheit 17 hinterlegt werden. Hiernach wird die Pumpvorrichtung 1 mit dem medizinischen Fluid 20 befüllt, so dass die elastomere Hohlmembran 3, wie anhand Fig. 3 ersichtlich ist, elastisch gedehnt wird. Infolge dieser elastischen Dehnung ändert sich die Länge der Hohlmembran 3 entlang der Längsrichtung L, wobei der Dehnungsmessstreifen 14 ebenfalls eine Längenänderung erfährt, infolge derer sich seine elektrische Leitfähigkeit verändert. In einem derart befülltem Zustand der Pumpvorrichtung 1 wird ein weiterer Referenzmesswert R2 ermittelt. Dieser Referenzmesswert R2 kann alternativ oder zusätzlich in der Speichereinheit 17 hinterlegt werden. Bei einer Entleerung des Pumpvolumens 9 reduziert sich die Dehnung der elastomeren Hohlmembran 3 und somit auch die Dehnung des Dehnungsmessstreifens 14 der Sensoranordnung 11. Ein bei einer solchen Entleerung ermittelter Dehnungsmesswert kann mittels der Auswerteeinheit 15 mit den in der Speichereinheit 17 hinterlegten Referenzmesswerten R1, R2 verglichen werden. Auf diese Weise ist letztlich eine aktuelle Füllstandsinformation F zum Füllzustand des Pumpvolumens 9 während des Entleerens ermittelbar. Diese Füllstandsinformation F kann einem Anwender der medizinischen Pumpvorrichtung 1 mittels der Anzeigeeinheit 18 der Ausgabeeinheit 16 optisch dargestellt werden. Alternativ oder zusätzlich kann die Füllstandsinformation F mittels der Übertragungseinheit 19 der Ausgabeeinheit 16 kabellos zu einer externen Anzeige- oder Wiedergabeeinheit übertragen werden.

## Patentansprüche

1. Medizinische Pumpvorrichtung (1) zur Förderung eines medizinischen Fluids (20) mit einem Grundkörper (2) und einer an dem Grundkörper (2) festgelegten elastomeren Hohlmembran (3), die derart angeordnet ist, dass zwischen dem Grundkörper (2) und der Hohlmembran (3) ein Pumpvolumen (9) zur Befüllung mit dem medizinischen Fluid (20) gebildet ist, wobei die Hohlmembran (3) in einem zumindest teilweise befüllten Füllzustand des Pumpvolumens (9) elastisch gedehnt ist und derart einen Förderdruck auf das Pumpvolumen (9) bewirkt, und mit einem Sensoriksystem (10), das zur Ermittlung der Dehnung (D) der Hohlmembran (3) vorbereitet ist, so dass unter Verwendung der derart ermittelten Dehnung (D) eine Füllstandsinformation (F) zum Füllzustand des Pumpvolumens (9) ermittelbar ist, **dadurch gekennzeichnet, dass** das Sensoriksystem (10) eine elektronische Auswerteeinheit (14) aufweist, die derart eingerichtet ist, dass ein mittels des Sensoriksystems (10) ermitteltes Messsignal (S) in einen Messwert (W) umsetzbar, ein Differenzenzwert (T) zwischen dem Messwert (W) und einem Referenzwert (R, R1, R2) ermittelbar und die Füllstandsinformation (F) unter Verwendung des Differenzwertes (T) ermittelbar ist.

2. Medizinische Pumpvorrichtung (1) nach Anspruch 1, **dadurch gekennzeichnet, dass** das Sensoriksystem (10) eine Sensoranordnung (11) aufweist, die an der Hohlmembran (3) angeordnet und mit dieser verbunden ist.

3. Medizinische Pumpvorrichtung (1) nach Anspruch 2, **dadurch gekennzeichnet, dass** die Sensoranordnung (11) und die Hohlmembran (3) mittels eines Koextrusionsverfahrens miteinander verbunden sind.

4. Medizinische Pumpvorrichtung (1) nach Anspruch 2 oder 3, **dadurch gekennzeichnet, dass** die Sensoranordnung (11) zumindest einen Dehnungsmessstreifen (14) aufweist.

5. Medizinische Pumpvorrichtung (1) nach einem der vorhergehenden Ansprüche, **dadurch gekennzeichnet, dass** das Sensoriksystem (10) eine Ausgabeeinheit (16) aufweist, die zur Ausgabe der Füllstandsinformation (F) eingerichtet ist.

6. Medizinische Pumpvorrichtung (1) nach Anspruch 5, **dadurch gekennzeichnet, dass** die Ausgabeeinheit (16) eine Anzeigeeinheit (18) aufweist, die zur optischen Darstellung der Füllstandsinformation (F) eingerichtet ist.

7. Medizinische Pumpvorrichtung (1) nach Anspruch 5 oder 6, **dadurch gekennzeichnet, dass** die Ausgabeeinheit (16) eine Übertragungseinheit (19) aufweist, die zur kabellosen Übertragung der Füllstandsinformation (F) eingerichtet ist.

## Claims

1. Medical pump device (1) for delivering a medical fluid (20), with a base body (2) and with an elastomeric hollow membrane (3) which is secured to the base body (2) and which is arranged in such a way that a pump volume (9) for filling with the medical fluid (20) is formed between the base body (2) and the hollow membrane (3), wherein the hollow membrane (3), in an at least partially filled state of the pump volume (9), is elastically extended and in this way exerts a delivery pressure on the pump volume (9), and with a sensor system (10) which is set up to determine the extension (D) of the hollow membrane (3) such that, by using the extension (D) that is thus determined, it is possible to determine filling level information (F) concerning the filling state of the pump volume (9),
**characterized in that**
the sensor system (10) has an electronic evaluation unit (14) which is configured in such a way that a measurement signal (S) determined by means of the sensor system (10) can be converted into a measurement value (W), a differential value (T) between the measurement value (W) and a reference value (R, R1, R2) can be determined, and the filling level information (F) can be determined using the differential value (T).

2. Medical pump device (1) according to Claim 1, **characterized in that** the sensor system (10) has a sensor array (11) which is arranged on the hollow membrane (3) and connected thereto.

3. Medical pump device (1) according to Claim 2, **characterized in that** the sensor array (11) and the hollow membrane (3) are connected to each other by a co-extrusion method.

4. Medical pump device (1) according to Claim 2 or 3, **characterized in that** the sensor array (11) has at least one strain gauge (14).

5. Medical pump device (1) according to any of the preceding claims, **characterized in that** the sensor system (10) has an output unit (16) which is configured to output the filling level information (F).

6. Medical pump device (1) according to Claim 5, **characterized in that** the output unit (16) has a display unit (18) which is configured to provide an optical presentation of the filling level information (F).

7. Medical pump device (1) according to Claim 5 or 6, **characterized in that** the output unit (16) has a transmission unit (19) which is configured for wireless transmission of the filling level information (F).

## Revendications

1. Dispositif de pompage médical (1) pour refouler un fluide médical (20), comprenant un corps de base (2) et une membrane creuse élastomère (3) fixée au corps de base (2), qui est disposée de telle sorte qu'entre le corps de base (2) et la membrane creuse (3) soit formé un volume de pompage (9) destiné à être rempli avec le fluide médical (20), la membrane creuse (3) étant étirée élastiquement dans un état de remplissage du volume de pompage (9) au moins partiellement rempli et provoquant ainsi une pression de refoulement sur le volume de pompage (9), et comprenant un système de capteur (10) qui est prévu pour détecter l'étirement (D) de la membrane creuse (3), de telle sorte qu'en utilisant l'étirement ainsi détecté (D), une information d'état de remplissage (F) relative à l'état de remplissage du volume de pompage (9) puisse être déterminée, **caractérisé en ce que** le système de capteur (10) présente une unité d'analyse électronique (14) qui est prévue de telle sorte qu'un signal de mesure (S) déterminé au moyen du système de capteur (10) puisse être converti en une valeur de mesure (W), qu'une valeur de différence (T) entre la valeur de mesure (W) et une valeur de référence (R, R1, R2) puisse être déterminée et que l'information d'état de remplissage (F) puisse être déterminée en utilisant la valeur de différence (T).

2. Dispositif de pompage médical (1) selon la revendication 1, **caractérisé en ce que** le système de capteur (10) présente un agencement de capteur (11) qui est disposé au niveau de la membrane creuse (3) et qui est connecté à celle-ci.

3. Dispositif de pompage médical (1) selon la revendication 2, **caractérisé en ce que** l'agencement de capteur (11) et la membrane creuse (3) sont connectés l'un à l'autre au moyen d'un procédé de coextrusion.

4. Dispositif de pompage médical (1) selon la revendication 2 ou 3, **caractérisé en ce que** l'agencement de capteur (11) présente au moins une jauge de contrainte (14).

5. Dispositif de pompage médical (1) selon l'une quelconque des revendications précédentes, **caractérisé en ce que** le système de capteur (10) présente une unité d'émission (16) qui est prévue pour émettre l'information d'état de remplissage (F) .

6. Dispositif de pompage médical (1) selon la revendication 5, **caractérisé en ce que** l'unité d'émission (16) présente une unité d'affichage (18) qui est prévue pour représenter optiquement l'information d'état de remplissage (F).

7. Dispositif de pompage médical (1) selon la revendication 5 ou 6, **caractérisé en ce que** l'unité d'émission (16) présente une unité de transmission (19) qui est prévue pour transmettre sans fil l'information d'état de remplissage (F).
